# FASCICULE DE BREVET EUROPEEN

(11) **EP 1 192 152 B1**
(45) Date de publication et mention de la délivrance du brevet: **27.11.2002**
(21) Numéro de dépôt: 00949575.5
(22) Date de dépôt: 29.06.2000
(51) Int. Cl.: C07D 405/04, A61K 31/4178, A61P 25/00

(54) **NOUVEAUX DERIVES BENZODIOXANNE IMIDAZOLINES FLUORES, LEUR PREPARATION ET LEURS APPLICATIONS EN THERAPEUTIQUE**
FLUORIERTE BENZODIOXAN IMIDAZOLIN DERIVATE, DEREN HERSTELLUNG UND DEREN THERAPEUTISCHE VERWENDUNG
NOVEL FLUORINATED IMIDAZOLINE BENZODIOXANE, PREPARATION AND THERAPEUTIC USES THEREOF

(30) Priorité: 29.06.1999 FR 9908302
(43) Date de publication de la demande: 03.04.2002
(73) Titulaire: PIERRE FABRE MEDICAMENT, 92100 Boulogne (FR)
(72) Inventeur: IMBERT, Thierry, F-81290 Viviers-les-Montagnes (FR); MAYER, Patrice, F-81100 Castres (FR); MARIEN, Marc, F-81100 Castres (FR); PAUWELS, Peters, F-81440 Lautrec (FR)
(74) Mandataire: Ahner, Francis
(86) Numéro de dépôt international: FR0001825
(87) Numéro de publication internationale: WO01000619

(56) Documents cités:
- EP-A- 0 092 328

## Description

La présente invention concerne les nouveaux dérivés benzodioxanne imidazolines fluorés répondant à la formule 1. 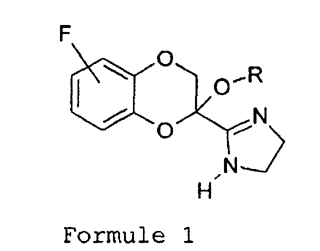 dans laquelle :
- R représente un groupe alcoyle ou alcényle, linéaire, ramifié ou cyclisé, comprenant 1 à 7 atomes de carbone, ou un groupe benzyle.
- L'atome de fluor sur l'homocycle peut occuper la position 5, 6, 7 ou 8.

Elle concerne les formes racémique et énantiomériquement pures, leurs formes salifiées ainsi que leur procédé de préparation.

Elle concerne aussi l'utilisation de ces composés à titre de médicament, ainsi que pour la préparation d'un médicament utilisé comme antagoniste des récepteurs α₂-adrénergiques et destinés à ce titre à traiter les maladies neurodégénératives, ainsi que leur progression.

De façon avantageuse, le radical R est un groupe méthyle, un groupe éthyle, un groupe n-propyle, un groupe iso-propyle, un groupe iso-butyle, un groupe cyclopropylméthyle, un groupe allyle, ou un groupe benzyle.

De préférence, l'atome de fluor occupe la position 5.

Il a été montré (Mavridis, Neuroscience (1991), 41, 507) que le locus coeruleus jouait un rôle prépondérant dans la récupération des fonctions dopaminergiques altérées par administration de MPTP chez le singe. Sa destruction entraînait une réduction de la récupération. Par ailleurs, des composés ayant une action α₂-antagoniste sont montrés comme réduisant les symptômes parkinsoniens chez le singe (Colpaert, Brain Res. Bull., 26, 627, 1991) ou chez le rat (Colpaert, Neuropharmacology, 26, 1431, 1987) par élévation de la libération de dopamine (Marien, M., Colpaert, F. Effect of (+)-efaroxan on mouse striatal dopamine metabolism in vivo. DOPAMINE 94-Satellite Meeting of the XIIth Int. Congr. Pharmacology, Quebec City, Canada, July 20-24, 1994).

De plus, un α₂ antagoniste, l'idazoxan, est montré comme ayant une action bénéfique sur les effets délétères de l'ischémie cérébrale (Gustafson, Exp. Brain Res., 86, 555, 1991 et J. Cereb. Blood Flow Metab., 1990, 10, 885) ainsi que dans la paralysie supranucléaire progressive, maladie neurodégénérative (Ghika, Neurology, 41, 986, 1991). Il a été également montré que des composés ayant une activité α₂ antagoniste provoquent une élévation de la libération d'acétylcholine au niveau du cortex préfrontal (Tellez, J.Neurochem. (1997), 68, 778).

Ainsi, une substance activant le système noradrénergique peut avoir la propriété de s'opposer à la progression de la dégénérescence des neurones impliqués, en réactivant les systèmes des différentes localisations cérébrales, qu'ils soient dopaminergiques, cholinergiques, ou que cela fasse appel à la libération de facteurs de croissance (Fawcett et al. J. Neurosci. (1998), 18, 2808-2821). Ces composés sont donc utiles dans les cas de maladies neurodégénératives- de type maladie de Parkinson ou d'Alzheimer et à leur progression, la chorée d'Huntington, la sclérose latérale amyotrophique, la maladie de Creutzfeld Jacob, la paralysie supranucléaire progressive, les troubles cognitifs et mnésiques, les déficits d'attention et de vigilance du sujet âgé, ainsi que la progression ou l'évolution de ces maladies ou de ces troubles. Sont concernés également les troubles ischémiques et post ischémiques cérébraux, les accidents vasculaires cérébraux et leurs conséquences, la dépression, la narcolepsie et les dysfonctionnements sexuels masculins, ainsi que les troubles liés au syndrome d'immunodéficience acquise.

Il est connu que des dérivés de benzodioxanne tels que l'idazoxan : 2-(1,4-benzodioxan-2-yl)-2-imidazoline, ou les alcoxy idazoxan : 2-(2-alcoxy-1,4-benzodioxan-2-yl)-2-imidazoline, possèdent des propriétés α₂ - antagonistes (J. Med. Chem. (1983), 26, 823 ; J. Med. Chem. (1985), 28, 1054). Ces composés ont été brevetés dans GB 2068376 pour l'idazoxan et dans EP 92328 pour les alcoxy-idazoxans.

Il a été montré dans ces publications qu'un grand nombre de dérivés de l'idazoxan ont été synthétisés et testés pour leur action agoniste ou antagoniste sur les récepteurs α₁ ou α₂, entre autres, les dérivés halogénés, substitués sur le noyau aromatique, tous ont été trouvés moins actifs, ou inactifs par rapport à leur analogue non substitué à l'idazoxan (en particulier le dérivé 6/7-fluoro, chloro ou bromo, le dérivé 5,8-dichloro, ou 8-chioro). Par ailleurs le dérivé du 2-méthoxy idazoxan subtitué en 6,7 par deux groupes méthoxy, n'a montré qu'une activité extrèmement marginale -en tant qu'antagoniste α₂ présynaptique.

Les composés de la présente invention se distinguent des composés connus par le fait qu'il possèdent un atome de fluor sur la position 5, 6, 7 ou 8 du noyau aromatique. Ils ont la propriété d'être des puissants antagonistes des récepteurs α₂-adrénergiques.

Il a été trouvé de façon remarquable que la présence de cet atome de fluor sur ces positions, confère à ces molécules des propriétés particulièrement intéressantes par rapport à leur analogue non fluoré.

Les propriétés pharmacologiques des produits de la présente invention ont été étudiées entre autre de façon comparatives avec celles du 2-méthoxy idazoxan (RX 821002) et du 2-éthoxy idazoxan (RX 811059), composés structurellement reliés, dérivés non substitués sur le noyau aromatique.

En effet, nous montrons, in vivo, la supériorité des propriétés pharmacologiques des produits de la présente invention, dans le test du déficit mnésique induit par la scopolamine, de l'antagonisme de l'hypothermie induite par le guanabenz, substance α₂ agoniste, et sur le niveau, dans le cortex, de la normétanéphrine, métabolite et marqueur sélectif de la libération de la noradrénaline.

Test du déficit mnésique induit par la scopolamine:

En accord avec l'hypothèse cholinergique des phénomènes d'apprentissage et de mémoire, la scopolamine possède des propriétés amnésiantes chez l'animal et l'homme. Son administration chez l'homme sain provoque certains symptômes d'amnésie proches de ce qui est observé dans la maladie d'Alzheimer. Il a été proposé que la scopolamine soit utilisée comme modèle pharmacologique expérimental de cette pathologie. Les similarités entre les déficits mnésiques de la maladie d'Alzheimer et ceux induits par la scopolamine chez le rat ont été publiées (P. Chopin et M. Briley, The effects of raubasine and dihydroergocristine on an age-related deficit in passive avoidance learning in rats, J.Pharm.Pharmacol. 42, 375-376, 1990). La scopolamine réduit la capacité d'acquisition, de mémorisation, et de rappel dans un test d'évitement passif chez le rat. Il s'agit de mesurer la réticence, après apprentissage, qu'a l'animal à entrer dans un compartiment sombre, où il reçoit un léger choc électrique. L'administration de scopolamine supprime cette réticence, et les composés étudiés s'opposent à l'effet de la scopolamine.

La comparaison des produits de la présente invention est faite avec le composé connu RX 821002 , énantiomère dextrogyre. Le protocole expérimental est celui publié par P. Chopin et M. Briley (Effects of four non-cholinergic cognitive enhancers in comparison with tacrine and galanthamine on scopolamine-induced amnesia in rats : Psychopharmacology, 106, 26-30, 1992).

Les résultats sont montrés dans le tableau suivant :

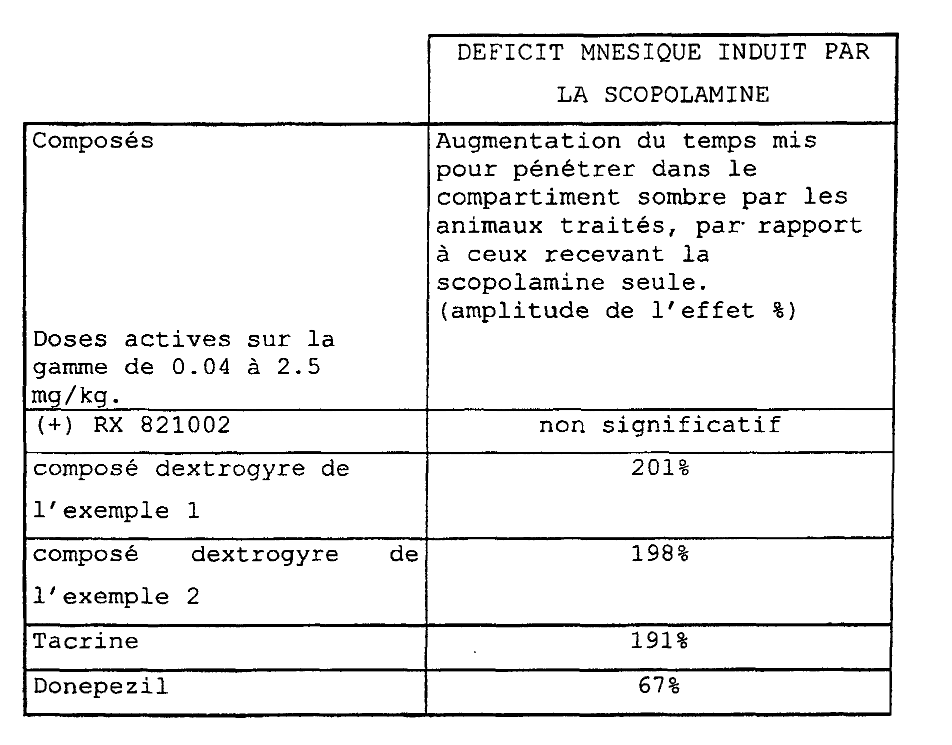

Les composés de la présente invention manifestent une activité importante sur une grande gamme de doses, contrairement au (+) RX 821002, qui n'est pas signicativement actif. L'amplitude de son effet est au moins aussi importante que celui de la tacrine, et plus actif que celui du donepezil , composés de référence utilisés en thérapeutique pour la maladie d'Alzheimer.

Il est ainsi montré l'intérêt et la différence importante des produits de l'invention.

Inhibition de l'hypothermie induite par le Guanabenz :

L'évaluation de l'activité biologique des composés de l'invention est également faite *in vivo* par l'étude de l'inhibition de l'hypothermie induite par un α₂-agoniste central comme le guanabenz selon le protocole de S.C. Dilsaver, Pharmacol. Biochem. Behav., 45, 247, 1993.

Ce test met en évidence l'effet antagoniste des récepteurs α₂-adrénergiques, *in vivo,* des composés de l'invention, ainsi que leur activité au niveau central. Les capacités inhibitrices sont exprimées en DE₅₀ qui représentent les doses produisant d'une part une inhibition significative contre l'hypothermie induite par le guanabenz, et d'autre part une normalisation, c'est à dire un retour à la température normale de l'animal avant injection de guanabenz. Ces valeurs sont obtenues en utilisant la méthode de J.T. Litchfield et F. Wilcoxon (J. Pharmacol. Exp. Ther., 96, 99, 1949). La comparaison est effectuée entre les composés 2-méthoxy, fluoré en position 5 (composé dextrogyre de l'exemple 1) et non fluoré : (+) RX 821002, et entre les composés 2-éthoxy, fluoré en position 5 (composé dextrogyre de l'exemple 2) et non fluoré : (+) RX 811059.

| | (+)RX 821002 | composé dextrogyre de l'exemple 1 |
|---|---|---|
| Gamme de doses actives (i.p) inhibition (mg/kg) DE₅₀ (mg/kg) | 0.01-10 0.01 | 0.0025-40 0.003 |
| normalisation (mg/kg) DE₅₀ (mg/kg) | 0.04-2.5 0.05 | 0.0025-10 0.02 |
| Gamme de doses actives (p.o.) normalisation (mg/kg) DE₅₀ (mg/kg) | 0.16-10 0.56 | 0.04-40 0.22 |

| | (+)RX 811059 | Composé dextrogyre de l'exemple 2 |
|---|---|---|
| Gamme de doses actives (i.p) Inhibition (mg/kg) DE₅₀ (mg/kg) | 0.04-10 0.08 | 0.0025-40 0.02 |
| normalisation (mg/kg) DE₅₀ (mg/kg) | 0.16-10 0.32 | 0.01-10 0.16 |

Il apparaît ainsi la plus grande puissance d'action des composés de l'invention par rapport à leurs analogues (+) RX 821002 et (+) RX 811059. Il est mis également en évidence l'amplitude de l'action, par la détermination de doses provoquant l'inhibition de l'hypothermie dans 100% des animaux, sur 6 doses (chaques doses séparées par un facteur de 4) pour le composé dextrogyre de l'exemple 1 contre 2 doses pour le (+) RX 821002. De même le composé dextrogyre de l'exemple 1 normalise l'hypothermie dans 100% des animaux sur 3 doses alors que le (+) RX 821002 ne le fait pas, si ce n'est que sur 80% des animaux et à 2 doses seulement.

Le tableau suivant montre ces résultats :

| | (+) RX 821002 | Composé dextrogyre de l'exemple 1 |
|---|---|---|
| Doses inhibant l'hypothermie dans 100% des animaux | 0.16 et 2.5 mg/kg | 0.01-0.04-0.16- 0.63-2.5-10 mg/kg |
| Doses normalisant l'hypothermie dans 100% des animaux | aucune | 0.16-0.63-2.5 mg/kg |

On voit ainsi que les produits de l'invention possèdent une gamme de doses actives très étendue, et se présente ainsi comme de meilleurs antagoniste α₂-adrénergique.

### Libération de noradrénaline :

Le taux de normétanéphrine, métabolite de la noradrénaline, dans les tissus cérébraux est utilisé comme mesure de la libération de la noradrénaline. Wood, P.L. et coll. Pharmacological Rev. 40, 163-187, (1988), et J. Neurochem. 48, 574-579, (1987)). La formation de la normétanéphrine, par l'action de la catéchol O-méthyltransférase a lieu à l'extérieur des neurones noradrénergiques et sa mesure rend compte des variations de libération de noradrénaline. Cette mesure est effectuée dans le cortex frontal, zone innervée principalement par le locus coeruleus.

Le composé à étudier est administré par voie i.p. à la souris, qui est sacrifiée après 60 min. par irradiation par micro-ondes (pour éviter les changements artéfactuels des taux de métabolites). Après dissection, la normétanéphrine est dosée par HPLC sur les extraits de tissus corticaux. Aux doses de 0.01 à 2.5 mg/kg la normétanéphrine dosée représente un taux de 125 à 150% supérieur avec le composé de l'exemple 1, par rapport au (+) RX 821002, dans les mêmes conditions. Ceci montre la plus grande efficacité de ce composé par rapport au (+) RX 821002 pour libérer la noradrénaline.

### Liaisons aux récepteurs α₂-adrénergiques in vitro :

Il a été vérifié également que les composés de l'invention possèdent une affinité sur les récepteurs α₂ adrénergiques humains, au niveau du nanomolaire sur la base des tests de binding sur les sous-types de ces récepteurs, en utilisant le 2-methoxy-idazoxan tritié, [³H] RX821002, comme ligand radioactif (J.C. Devedjian et coll. Eur.J.Pharmacol. (1994), 252, 43-49).

Les tests *in vivo* montrent l'avantage que peut apporter une substitution par un atome de fluor sur le noyau aromatique par rapport au composé dénué- de cette substitution.

Les composés de la présente invention possédant un carbone asymétrique, ils se présentent sous une forme dextrogyre et une forme lévogyre. La présente invention concerne donc également les composés énantiomériquement purs, leurs sels d'addition ainsi que les compositions pharmaceutiques comprenant au moins un composé de formule 1 et un excipient approprié. Les compositions pharmaceutiques peuvent être présentées de façon adaptée, pour l'administration par voie orale, injectable ou parentérale, sous forme de capsules, de gélules, de comprimés, ou de préparations injectables, à la dose journalière de 0,1 à 200 mg.

Les composés de la présente invention peuvent être préparés à partir du 3-fluorocatéchol, (décrit dans J.Amer.Chem.Soc. 77, 5314-5317, 1955) par condensation avec le 2,3-dibromopropionamide dans l'acétonitrile en présence de K₂CO₃ pour fournir les 2 régioisomères les 5 et 8-fluoro-1,4-benzodioxanne-2-carboxamide. Plusieurs recristallisations permettent d'isoler le dérivé 5-fluoro à l'état pur, au dépend du dérivé 8-fluoro qui se trouve plus soluble dans ces conditions. La fonction amide en position 2 du benzodioxanne est deshydratée en nitrile. Ce nitrile est alors bromé par action de la NBS pour obtenir le bromonitrile, lequel est soumis à l'action d'un alcoolate de sodium comme le méthanolate de sodium dans le méthanol, pour former l'imidate intermédiaire qui réagit *in situ* avec l'éthylènediamine pour former le dérivé α-méthoxy-imidazoline recherché. De la même façon, on accède aux différents alcoxy en position 2, par traitement du dérivé bromonitrile précédent par traitement avec un alcoolate alcalin correspondant.

La séparation des 2 énantiomères peut se faire de plusieurs façons: soit par cristallisation diastéréosélective avec un acide chiral qui peut-être l'acide tartrique ou ses dérivés comme l'acide dibenzoyltartrique soit par séparation chromatographique, HPLC préparative, sur phase chirale, fournissant l'isomère dextrogyre et l'isomère levogyre dont le chlorhydrate peut s'obtenir de façon cristalline par les méthodes usuelles.

Les dérivés 6 et 7 fluoro sur le cycle aromatique s'obtiennent à partir du (6 ou 7-fluoro-2,3-dihydrobenzo[1,4]dioxin-2-yl)-méthanol, décrit dans J. Med. Chem. (1987), 30, 814. Ces dérivés méthanol sont oxydés en acide puis amidifiés et dehydratés en nitrile selon les procédés décrits dans J. Med. Chem. (1983), 26, 823, ou J. Med. Chem. (1985), 28, 1054, puis traités comme indiqué précédemment.

Les modes opératoires des différents stades de la synthèse permettent d'illustrer l'invention :

### EXEMPLE 1 : 2-(5-Fluoro-2-méthoxy-1,4-benzodioxan-2-yl)-2-imidazoline

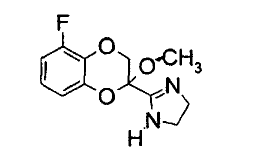

### Stade 1 : 5-Fluoro-1,4-benzodioxanne-2-carboxamide.

Une solution contenant 50 g de 3-fluorocatéchol (391 mmol), 99,3g de 2,3-dibromopropionamide (430 mmol, 1,1 éq) et 108,1 g de carbonate de potassium pulvérisé (782 mmol, 2 éq) dans 400 ml d'acétonitrile est chauffée à 60°C pendant 16 h. Le mélange réactionnel est filtré puis le filtrat est évaporé à sec. On obtient 70,5 g d'un solide jaune clair (rendement 92%; mélange 1/1 des deux régioisomères). Des recristallisations successives dans l'éthanol à chaud donnent 16,7 g de 5-fluoro-1,4-benzodioxanne-2-carboxamide pur (rendement 22%).
T°fusion : 167°C.
RMN ¹H (400MHz, CDCl₃) :6,86-6,71 (m, 3H, aromatiques); 6,52 (s large, 1H, NH); 6,11 (s large, 1H, NH); 4,72 (dd, J=2,4 et 7,2 Hz, 1H, H2); 4,62 (dd, J=2,4 et 11,6 Hz, 1H, H3A); 4,23 (dd, J=7,2 et 11,6 Hz, 1H, H3B).

### Stade 2 : 5-Fluoro-1,4-benzodioxanne-2-carbonitrile

A une suspension de 16,7 g d'amide du stade 1 (84,5 mmol) dans 180 ml de dioxanne à 0°C, on ajoute 13,7 ml de pyridine (169 mmol, 2 éq), puis après 10 min. on ajoute, goutte à goutte, 13,1ml d'anhydride trifluoroacétique (19,5 g; 93 mmol, 1,1 éq). Le mélange réactionnel est maintenu à froid pendant 1h. puis est agité à température ambiante pendant 16 h. La solution est reprise par Et₂O/HCl 1N. La phase organique est lavée par NaOH 1N, séchée sur MgSO₄, filtrée puis évaporée à sec. On obtient 16,8 g d'une huile jaune claire (rendement quantitatif).
RMN ¹H (400MHz, CDCl₃) : 6,90-6,73 (m, 3H, aromatiques); 5,15 (dd, J=3,6 et 2,4 Hz, 1H, H2); 4,50 (dd, J=11,6 et 3,6 Hz, 1H, H3A); 4,41 (dd, J=11,6 et 2,4 Hz, 1H, H3B).

### Stade 3 : 5-Fluoro-2-bromo-1,4-benzodioxanne-2-carbonitrile

Une solution contenant 6,44 g de nitrile obtenu au stade 2 (36 mmol), 6,40 g de NBS (36 mmol, 1 éq), 100 mg de peroxyde de benzoyle dans 200 ml de CCl₄ est chauffée à 70°C. pendant 48 h. On laisse refroidir le mélange à température ambiante. Le mélange réactionnel est alors filtré. Le solide est lavé par CCl₄ et les eaux mères sont évaporées à sec. On isole 9,3 g d'une huile jaune orangée (rendement quantitatif).
RMN ¹H (400MHz, CDCl₃) : 6,93 (m, 2H, aromatiques); 6,81 (m, 1H, aromatique); 4,62 (d, J=11,6 Hz, 1H, H3A); 4,48 (d, J=11,6 Hz, 1H, H3B).

### Stade 4 : 2-(5-Fluoro-2-méthoxy-1,4-benzodioxan-2-yl)-2-imidazoline

Une solution contenant 7 g de 2-bromo-5-fluoro-1,4-benzodioxanne-2-carbonitrile (27,1 mmol), 220 mg de méthylate de sodium (4 mmol, 0,15 éq) dans 150 ml de méthanol sont agités à température ambiante pendant ¾ h. On ajoute alors 2 ml d'éthylènediamine (1,79g, 29,8 mmol, 1,1 éq) puis 11,3 ml d'une solution HCl/iPrOH 3N (34 mmol, 1,25 éq). Le mélange réactionnel est agité à température ambiante pendant 16 h, puis repris par un mélange NaOH 1N/CH₂Cl₂. La phase organique est séchée sur MgSO₄, filtrée puis évaporée à sec. Le produit brut est purifié par chromatographie sur silice sous pression (CH₂Cl₂/MeOH 96/4). On obtient 3,9 g de produit pur (rendement 57%).
T°fusion : 134°C.
RMN ¹H (400MHz, CDCl₃) : 6,86-6,74 (m, 3H, aromatiques); 5,14 (s large, 1H, NH); 4,57 (d, J=11,2 Hz, 1H, H3A); 4,00 (d, J=11,2 Hz, 1H, H3B); 3,75 (massif très large, 4H, imidazoline); 3,39 (s, 3H, OCH₃).

Le chlorhydrate est obtenu par dissolution de 500 mg de base dans l'éther puis addition de 661 ml d'une solution HCl/iPrOH 3N. Le solide formé est filtré, lavé à l'éther puis séché sous vide. On obtient 480 mg de sel.
T°fusion > 260°C.

| analyse élémentaire: | | | |
|---|---|---|---|
| théorique | C(49,40) | H(4,89) | N(9,70); |
| expérimentale | C(49,42) | H(4,91) | N(9,61) |

Le composé racémique en tant que base (2 g) est chromatographié par injections successives d'une quantité de l'ordre de 500 à 800 mg, sur un colonne préparative HPLC Prochrom diam.50mm (Chiralpack AD), et par élution avec le mélange : hexane / isopropanol / diéthylamine : 85 /15/0.001. Par un débit de 100 ml/mn, on isole successivement les énantiomères dextrogyres et lévogyres de l'exemple. Les chlorhydrates des énantiomères sont précipités dans l'éther par addition d'une quantité stoéchiométrique d'éthanol saturé de gaz chlorhydrique.

En particulier :
Enantiomère (+):
[α_{D}]^{23°} = + 90.8° (c = 0.58, MeOH).
T° fusion : sublimation à partir de230°C.

| analyse élémentaire (C₁₂H₁₃N₂O₃F, HCl) : | | | |
|---|---|---|---|
| théorique | C(49,92) | H(4,89) | N(9,70) ; |
| expérimentale | C(49,94) | H(4,77) | N(9,57). |

Enantiomère (-) :
[α_{D}]^{23°} = - 93.9° (c = 0.43, MeOH).
T° fusion : sublimation à partir de 230°C.

| analyse élémentaire (C₁₂H₁₃N₂O₃F, HCl) : | | | |
|---|---|---|---|
| théorique | C(49,92) | H(4,89) | N(9,70); |
| expérimentale | C(49,89) | H(4,83) | N(9,61). |

### EXEMPLE 2 : 2-(5-Fluoro-2-éthoxy-1,4-benzodioxan-2-yl)-2-imidazoline.

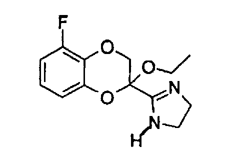

On dissout 115 mg de sodium dans 200 ml d'éthanol, puis on ajoute 8,52 g de 2-bromo-5-fluoro-1,4-benzodioxanne-2-carbonitrile (33 mmol ), obtenu au stade 3 de l'exemple 1, et on agite à température ambiante pendant ¾ heure. On ajoute alors 2,43 ml d'éthylènediamine (2,18 g; 36,3 mmol; 1,1 éq. mol.) puis 13,8 ml d'une solution HCl/iPrOH 3N (41,3 mmol. 1,25 éq. mol.). Le mélange réactionnel est agité à température ambiante pendant 16 h. puis repris par un mélange NaOH 1N/CH₂Cl₂. La phase organique est séchée sur MgSO₄, filtrée puis évaporée à sec. Le produit brut est purifié par chromatographie sur silice sous pression (CH₂Cl₂/MeOH 96/4 ). On obtient 4,17 g de produit pur (rendement 48%).
RMN ¹H (400MHz, CDCl₃) : 6,80 (m, 3H, aromatiques); 5,14 (s large, 1H, NH); 4,53 (d, J=11,2 Hz, 1H, H3A); 4,10 (d, J=11,2 Hz, 1H, H3B ); 3,97-3,45 massif très large, 4H, imidazoline ); 3,70 (m, 2H, OCH₂ ); 1,12 (t, J=7,2 Hz, 3H, CH₃) .

La séparation des deux énantiomères se fait par HPLC chirale (colonne Chiralpack AD; Hexane/iPrOH/diéthylamine 96/4/0,1; 100 ml/mn; 230 nm).

Les chlorhydrates sont obtenus par dissolution de la base dans l'éther puis addition d'une solution HCl/iPrOH 3N. Le solide formé est filtré, lavé à l'éther puis séché sous vide.
Enantiomère (+) :
[α_{D}]²⁴ (c=0,380; MeOH) = +80,9°.
T° fusion > 260°C.

| analyse élémentaire (C₁₃H₁₅N₂O₃F, HCl) : | | | |
|---|---|---|---|
| théorique | C(51,58) | H(5,33) | N(9,25) |
| expérimentale | C(51,24) | H(5,36) | N(8,94) |

Enantiomère (-) :
[α_{D}]²⁴ (c=0,380; MeOH) = -77,6°.
T° fusion > 260°C.

| analyse élémentaire (C₁₃H₁₅N₂O₃F, HCl) : | | | |
|---|---|---|---|
| théorique | C(51,58) | H(5,33) | N(9,25); |
| expérimentale | C(51,80) | H(5,39) | N(9,02). |

Les composés suivants sont obtenus suivant les mêmes modes opératoires que ceux décrits ci dessus :

### EXEMPLE 3 : 2-(5-fluoro-2-propoxy-1,4-benzodioxanne-2-yl)-imidazoline, chlorhydrate

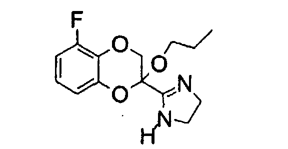

RMN ¹H (400MHz, DMSO d6) : 11,02 (s, 2H, NH,HCl); 6,99 (m, 2H, aromatiques); 6,92 (m, 1H, aromatique); 4,63 (d, J=11,5 Hz, 1H, H3A ); 4,23 (d, J=11,5 Hz, 1H, H3B ); 3,99 (s, 4H, imidazoline ); 3,55 ( m, 2H, OCH₂ ); 1,44 (m, 2H, OCH₂CH₂ ); 1,12 (t, J=7,2 Hz, 3H, CH₃) .
T° fusion : 206°C.

| analyse élémentaire (C₁₄H₁₇N₂O₃F, HCl) : | | | |
|---|---|---|---|
| théorique | C(53,09) | H(5,73) | N(8,84); |
| expérimentale | C(52,29) | H(5,82) | N(8,63). |

### EXEMPLE 4 : 2-(5-fluoro-2-isopropoxy-1,4-benzodioxanne-2-yl)-imidazoline, chlorhydrate

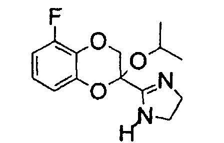

RMN ¹H (400MHz, DMSO d6): 10,99 (s, 2H, NH,HCl); 6,99 (m, 2H, aromatiques); 6,90 (m, 1H, aromatique); 4,59 ( d, J=11,6 Hz, 1H, H3A ); 4,18 ((d, J=11,6 Hz, 1H, H3B ); 4,08 ( m, 1H, OCH); 3,99 ( s, 4H, imidazoline ); 1,18 (d, J=6 Hz, 3H, CH₃); 0,96 (d, J=6 Hz, 3H, CH₃).

| analyse élémentaire (C₁₄H₁₇N₂O₃F, HCl, ½ H₂O) : | | | |
|---|---|---|---|
| théorique | C(51,55) | H(5,82) | N(8,30); |
| expérimentale | C(51,62) | H(5,88) | N(8,60). |

### EXEMPLE 5 : 2-(5-fluoro-2-isobutoxy-1,4-benzodioxanne-2-yl)-imidazoline, chlorhydrate

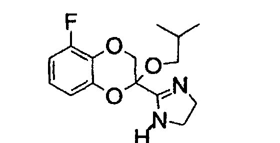

RMN ¹H (400MHz, DMSO d6 ) : 10,79 (s, 2H, NH,HCl); 6,97 m, 2H, aromatiques ); 6,90 (m, 1H, aromatique); 4,61 ( d, J=11,4 Hz, 1H, H3A ); 4,22 (d, J=11,4 Hz, 1H, H3B); 3,97 (s, 4H, imidazoline); 3,37 (m, 2H, OCH₂) ; 1,70 (m, 1H, CH ); 0,75 (d, J=6,8 Hz, 3H, CH₃ ); 0,65 (d, J=6,8 Hz, 3H, CH₃).
T° fusion : 206°C.

| analyse élémentaire (C₁₅H₁₉N₂O₃F, HCl) : | | | |
|---|---|---|---|
| théorique | C(54,47) | H(6,09) | N(8,47); |
| expérimentale | C(53,83) | H(6,36) | N(8,27). |

### EXEMPLE 6 : 2-(5-Fluoro-2-cyclopropylméthyloxy-1,4-benzodioxanne-2-yl)-imidazoline, chlorhydrate

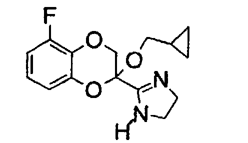

RMN ¹H (400MHz, DMSO d6): 10,98 (s, 2H, NH,HCl) ; 6,99 (m, 2H, aromatiques); 6,90 (m,1H,aromatique); 4,61 (d,J=11,5Hz,1H,H3A; 4,22 (d,J=11,5Hz,1H,H3B) ; 3,98 (s,4H, imidazolin); 3,44 (m,2H,OCH₂) ; 0,93(m,1H,CH) ; 0,42 (m,2H,cyclopropyl) ; 0,18 (m,1H,cyclopropyl) ; 0,00 (m,1H,cyclopropyl).

| analyse élémentaire (C₁₅H₁₇N₂O₃F, HCl) : | | | |
|---|---|---|---|
| théorique | C(54,80) | H(5,52) | N(8,52) ; |
| expérimentale | C(54,09) | H(5,23) | N(8,33). |

### EXEMPLE 7 : 2-(5-Fluoro-2-allyloxy-1,4-benzodioxanne-2-yl)-imidazoline, chlorhydrate

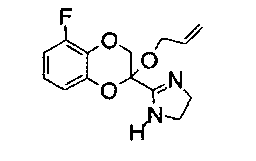

RMN ¹H (400MHz, DMSO d6) : 11,10 (s, 2H, NH,HCl); 6,99 (m, 2H, aromatiques ); 6,92 (m, 1H, aromatique); 5,78 (m, 1H, CH=CH₂); 5,25 (d, J=17,2 Hz, 1H, CH=CH₂) ; 5,13 (d, J=10,4 Hz, 1H, CH=CH₂) 4,68 (d, J=11,6 Hz, 1H, H3A); 4,26 (d, J=11,6 Hz, 1H, H3B); 4,07 (m, 2H, OCH₂) ; 3,98 (s, 4H, imidazoline).
T° fusion : 214°C.

| analyse élémentaire (C₁₄H₁₅N₂O₃F, HCl) : | | | |
|---|---|---|---|
| théorique | C(53,43) | H(5,12) | N(8,90); |
| expérimentale | C(52,86) | H(5,23) | N(8,81). |

### EXEMPLE 8 : 2-(5-Fluoro-2-benzyloxy-1,4-benzodioxanne-2-yl)-imidazoline, chlorhydrate

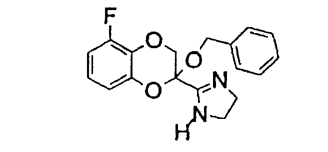

RMN ¹H (400MHz, DMSO d6) : 11,15 (s, 2H, NH,HCl); 7,30 (m, 3H, aromatiques); 7,23 (m, 2H, aromatiques); 6,99 ( m, 2H, aromatiques ); 6,91 (m, 1H, aromatique); 4,70 (m, 3H, H3A et PhCH₂); 4,29 (d, J=11,6 Hz, 1H, H3B); 3,96 (s, 4H, imidazoline).
T° fusion : 218°C.

| analyse élémentaire (C₁₈H₁₇N₂O₃F, HCl, H₂O) : | | | |
|---|---|---|---|
| théorique | C(56,48) | H(5,27) | N(7,32); |
| expérimentale | C(56,50) | H(5,31) | N(7,21). |

### EXEMPLE 9 : 2-(6-Fluoro-2-methoxy-1,4-benzodioxanne-2-yl)-imidazoline, chlorhydrate

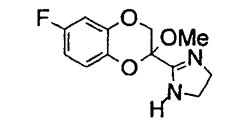

Ce composé est préparé via le 2-(6-fluoro-2-methoxy-1,4-benzodioxanne-2-yl)méthanol décrit dans J.Med.Chem. (1987), 30, 814, puis transformé en imidazoline selon J.Med.Chem. (1983) , 26, 823, ou J.Med.Chem. (1985), 28, 1054. Analyse élémentaire : (C₁₂H₁₃N₂O₃F, HCl).

### EXEMPLE 10 : 2-(7-Fluoro-2-méthoxy-1,4-benzodioxanne-2-yl)-imidazoline, chlorhydrate :

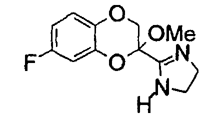

Ce composé est préparé via le 2-(7-fluoro-2-methoxy-1,4-benzodioxanne-2-yl)méthanol décrit dans J.Med.Chem. (1987), 30, 814, puis transformé en imidazoline selon J.Med.Chem. (1983) , 26, 823, ou J.Med.Chem. (1985), 28, 1054. Analyse élémentaire : (C₁₂H₁₃N₂O₃F, HCl).

### EXEMPLE 11 : 2-(8-Fluoro-2-méthoxy-1,4-benzodioxanne-2-yl)-imidazoline, chlorhydrate

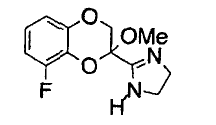

### Stade 1 : 5/8-Fluoro-1,4-benzodioxanne-2-carbonitrile :

On ajoute goutte à goutte 15,8 ml d'anhydride trifluoroacétique (23,4 g, 0,11 mol) à une solution contenant 20 g de 5/8-flurobenzodioxanne-2-carboxamide, obtenu au stade 1 de l'exemple 1, et 16,4 ml de pyridine (16,1 g, 0,2 mol) dans 20 ml de dioxanne maintenue à 0°C dans un bain de glace. La réaction est maintenue à froid pendant 2 h, puis est extraite par Et₂O / HCl 1N. La phase acide est lavée 3 x par de l'éther. Les phases éthérées sont séchées sur MgSO₄, filtrées puis évaporées à sec. On obtient 16,34 g d'un mélange brut qui est réengagé dans l'étape suivante sans purification supplémentaire.

### Stade 2 : 2-Bromo-8-fluoro-1,4-benzodioxanne-2-carbonitrile :

Une solution contenant 16,34 g de 5/8-fluoro-1,4-benzodioxanne-2-carbonitrile (91,3 mmoles), 17,87 g de N-bromosuccinimide (100 mmoles) et 200 mg de peroxyde de benzoyle dans 500 ml de tétrachlorure de carbone est chauffée au reflux pendant 5 jours. Le mélange réactionnel est refroidi à température ambiante, puis le précipité de succinimide est éliminé par filtration. Le filtrat est évaporé à sec pour donner 22,2 g de mélange brut contenant le 2-bromo-5-fluoro-1,4-benzodioxanne-2-carbonitrile et le 2-bromo-8-fluoro-1,4-benzodioxanne-2-carbonitrile. Les deux isomères sont séparés par chromatographie flash sur colonne de silice (éther de pétrole / acétate d'éthyle 99,5/0,5). On obtient 7 g de 2-bromo-5-fluoro-1,4-benzodioxanne-2-carbonitrile et 6,2 g de 2-bromo-8-fluoro-1,4-benzodioxanne-2-carbonitrile recherché.
2-bromo-8-fluoro-1,4-benzodioxanne-2-carbonitrile :
RMN ¹H ( 400MHz, CDCl₃ ) : 7,02 ( m, 1H, aromatique ); 6,83 ( m, 2H, aromatiques ); 4,58 ( d, J=11,6Hz, 1H, H3A ) ; 4,48 ( d, J=11,6Hz, 1H, H3B ).

### Stade 3 : 2-(8-Fluoro-2-méthoxy-1,4-benzodioxanne-2-yl)-imidazoline

Une solution contenant 1,6 g de 2-bromo-8-fluoro-1,4-benzodioxanne-2-carbonitrile ( 6,2 mmol ), 40 mg de méthylate de sodium (0,7 mmol; 0,12 éq. mol. ) dans 50 ml de méthanol sont agités à température ambiante pendant ¾ heures. On ajoute alors 0,456 ml d'éthylènediamine (0,41 g; 6,8 mmol; 1,1 éq. mol. ) puis 2,3 ml d'une solution HCl/iPrOH 3N ( 6,8 mmol; 1,1 éq. mol.). Le mélange réactionnel est agité à température ambiante pendant 16 h. puis repris par un mélange NaOH 1N/CH₂Cl₂. La phase organique est séchée sur MgSO₄, filtrée puis évaporée à sec. Le produit brut est purifié par chromatographie sur silice sous pression ( CH₂Cl₂/MeOH 96/4 ). On obtient 0,75 g de produit pur ( rendement 48% ).
RMN ¹H ( 400MHz, CDCl₃) : 6,85 (m, 1H, aromatique); 6,73(m, 2H, aromatiques ); 4,57 ( d, J=11,6 Hz, 1H, H3A ); 4,00 ( d, J=11,6 Hz, 1H, H3B ); 3,76 ( massif large, 4H, imidazoline ); 3,42 ( s, 3H, OCH₃ ).
RMN ¹³C (100,03 Hz, CDCl₃) : 162,34 (C quat. imidazoline), 151,99 (d, J= 244 Hz, C8), 144,31 (C4a), 128,90 (d, J=14 Hz, C8a), 121,23 (d, J=9 Hz, C6), 112,51 (d, J=3,7 Hz, C5), 108,79 (d, J=18 Hz, C7), 94,15 (C2), 67,93 (C3), 51,58 (OCH₃), 50,5 (massif très large, CH₂ imidazoline).

Le chlorhydrate est obtenu par dissolution de la base dans l'éther puis addition d'un équivalent d'une solution HC1/iPrOH 3N. Le solide formé est filtré, lavé à l'éther puis séché sous vide.

Analyse élémentaire: théorique C(49,92) H(4,89) N(9,70);
expérimentale: C(49,63) H(4,93) N(9,54).

Les énantiomères bases sont séparés par HPLC chirale (colonne Chiralpack AD; Hexane/iPrOH/diéthylamine 90/10/0,1; 100 ml/mn; 254 nm).

Les chlorhydrates sont obtenus par dissolution de la base dans l'éther puis addition de l'équivalent d'une solution HCl/iPrOH 3N. Le solide formé est filtré, lavé à l'éther puis séché sous vide.
Enantiomère (+) :
[α_{D}]²⁵ (c=0,253; MeOH) = +86,2°.
T° fusion =262°C.

| analyse élémentaire (C₁₃H₁₅N₂O₃F₁, HCl) : | | | |
|---|---|---|---|
| théorique | C(49,92) | H(4,89) | N(9,70); |
| expérimentale | C(49,70) | H(4,87) | N(9,56). |

Enantiomère (-) :
[α_{D}]²⁵ (c=0,429; MeOH) = -85,8°.
T° fusion = 260°C.

| analyse élémentaire (C₁₃H₁₅N₂O₃F₁, HCl) : | | | |
|---|---|---|---|
| théorique | C(49,92) | H(4,89) | N(9,70); |
| expérimentale | C(49,55) | H(4,83) | N(9,57). |

L'invention s'étend également à l'utilisation des composés de formule 1 pour la préparation d'un médicament comme antagoniste des récepteurs adrénergiques α₂ utilisé et destiné à ce titre à traiter les maladies neurodégénératives et leur progression, les troubles cognitifs et mnésiques, ainsi que les déficits d'attention et de vigilance, la maladie d'Alzheimer, la maladie de Parkinson, la chorée d'Huntington, la sclérose latérale amyotrophique, la maladie de Creutzfeld Jacob, la paralysie supranucléaire progressive, ainsi que l'évolution de ces maladies ou de ces troubles. Sont également concernés les attaques cérébrales, les troubles ischémiques et post ischémiques cérébraux, la dépression, la narcolepsie et les dysfonctionnements sexuels masculins, ainsi que les troubles liés au syndrome d'immunodéficience acquise. Sont enfin concernés les pathologies relatives aux attaques cérébrales, aux troubles ischémiques, aux accidents vasculaires cérébraux et leurs conséquences, ainsi que la dépression, la narrolepsie, les dysfonctionnements sexuels masculins, les troubles liés au syndrome d'immunodéficience acquise, ainsi que l'évolution de ces maladies ou de ces troubles.

## Revendications

1. Composés correspondant à la structure de la formule générale 1 : 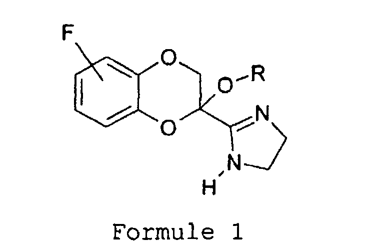 dans laquelle :
- R représente un groupe alcoyle ou alcényle, linéaire, ramifié ou cyclisé, comprenant 1 à 7 atomes de carbone, ou un groupe benzyle, et
- l'atome de fluor peut occuper la position 5, 6, 7 ou 8,
sous leur forme racémique et leurs formes énantiomériques pures, dextrogyres et lévogyres, ainsi que leurs sels d'addition.

2. Composés de formule 1 selon la revendication 1, **caractérisés en ce que** le radical R est un groupe méthyle.

3. Composés de formule 1 selon la revendication 1, **caractérisés en ce que** le radical R est un groupe éthyle.

4. Composés de formule 1 selon la revendication 1, **caractérisés en ce que** le radical R est un groupe *n*-propyle.

5. Composés de formule 1 selon la revendication 1, **caractérisés en ce que** le radical R est un groupe *iso*-propyle.

6. Composés de formule 1 selon la revendication 1, **caractérisés en ce que** le radical R est un groupe *iso*-butyle.

7. Composés de formule 1 selon la revendication 1, **caractérisés en ce que** le radical R est un groupe cyclopropylméthyle.

8. Composés de formule 1 selon la revendication 1, **caractérisés en ce que** le radical R est un groupe allyle.

9. Composés de formule 1 selon la revendication 1, **caractérisés en ce que** le radical R est un groupe benzyle.

10. Composés selon l'une des revendications 1 à 9, **caractérisés en ce que** l'atome de fluor occupe la position 5.

11. Un procédé de préparation des composés de formule 1 selon la revendication 1, **caractérisé en ce que** l'on fait réagir le 3-fluorocatéchol avec le 2,3-dibromo propionamide, et que l'on cristallise le dérivé 5-fluorobenzodioxanne-2-carboxamide obtenu, qui est ensuite déshydraté en nitrile, puis bromé par la NBS, soumis à un traitement par un alcoolate de sodium pour former l'imidate intermédiaire, qui est ensuite traité par l'éthylène diamine dans un alcool.

12. Un procédé de préparation des composés de formule 1 selon la revendication 1, **caractérisé en ce que** l'on transforme le 6 ou 7-fluoro-2,3-dihydro-benzo[1,4]dioxin-2-yl)-méthanol en nitrile, et que l'on traite ce nitrile selon le procédé de la revendication 11.

13. A titre de médicament, un composé de formule 1 selon l'une des revendications 1 à 10.

14. Compositions pharmaceutiques **caractérisées en ce qu'**elles comprennent au moins un composé de formule 1 selon les revendications 1 à 10, et un excipient approprié.

15. Utilisation d'un composé de formule 1 selon l'une des revendications 1 à 10 pour la préparation d'un médicament comme antagoniste des récepteurs adrénergiques α₂ utilisé et destiné à ce titre à traiter les maladies neurodégénératives et leur progression.

16. Utilisation d'un composé de formule 1 selon l'une des revendications 1 à 10 pour la préparation d'un médicament comme antagoniste des récepteurs adrénergiques **α**₂ utilisé et destiné à ce titre à traiter les troubles cognitifs et mnésiques, ainsi que les déficits d'attention et de vigilance.

17. Utilisation d'un composé de formule 1 selon l'une des revendications 1 à 10 pour la préparation d'un médicament comme antagoniste des récepteurs adrénergiques α₂ utilisé et destiné à ce titre à traiter la maladie d'Alzheimer.

18. Utilisation d'un composé de formule 1 selon l'une des revendications 1 à 10 pour la préparation d'un médicament comme antagoniste des récepteurs adrénergiques α₂ utilisé et destiné à ce titre à traiter la maladie de Parkinson, la chorée d'Huntington, la sclérose latérale amyotrophique, la maladie de Creutzfeld Jacob, la paralysie supranucléaire progressive, ainsi que l'évolution de ces maladies ou de ces troubles.

19. Utilisation d'un composé de formule 1 selon l'une des revendications 1 à 10, pour la préparation d'un médicament comme antagoniste des récepteurs adrenergétiques α₂ utilisé et destiné à ce titre à traiter les pathologies relatives aux attaques cérébrales, aux troubles ischémiques, aux accidents vasculaires cérébraux et leurs conséquences, ainsi que la dépression, la narrolepsie, les dysfonctionnements sexuels masculins, les troubles liés au syndrome d'immunodéficience acquise, ainsi que l'évolution de ces maladies ou de ces troubles.

## Patentansprüche

1. Verbindungen, die der Struktur der allgemeinen Formel 1 entsprechen: 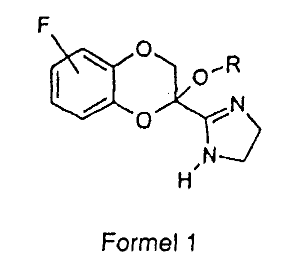 in der:
- R eine lineare, verzweigte oder cyclische Alkyl- oder Alkenylgruppe, die 1 bis 7 Kohlenstoffatome umfasst, oder eine Benzylgruppe darstellt, und
- das Fluoratom die Position 5, 6, 7 oder 8 einnehmen kann,
in ihrer racemischen Form und ihren reinen rechtsdrehenden oder linksdrehenden enantiomeren Formen sowie ihre Additionssalze

2. Verbindungen der Formel 1 gemäß Anspruch 1, **dadurch gekennzeichnet, dass** der Rest R eine Methylgruppe ist.

3. Verbindungen der Formel 1 nach Anspruch 1, **dadurch gekennzeichnet, dass** der Rest R eine Ethylgruppe ist.

4. Verbindungen der Formel 1 nach Anspruch 1, **dadurch gekennzeichnet, dass** der Rest R eine n-Propylgruppe ist.

5. Verbindungen der Formel 1 nach Anspruch 1, **dadurch gekennzeichnet, dass** der Rest R eine lsopropylgruppe ist.

6. Verbindungen der Formel 1 nach Anspruch 1, **dadurch gekennzeichnet, dass** der Rest R eine Isobutylgruppe ist.

7. Verbindungen der Formel 1 nach Anspruch 1, **dadurch gekennzeichnet, dass** der Rest R eine Cyclopropylmethylgruppe ist.

8. Verbindungen der Formel 1 nach Anspruch 1, **dadurch gekennzeichnet, dass** der Rest R eine Allylgruppe ist.

9. Verbindungen der Formel 1 nach Anspruch 1, **dadurch gekennzeichnet, dass** der Rest R eine Benzylgruppe ist.

10. Verbindungen nach einem der Ansprüche 1 bis 9, **dadurch gekennzeichnet, dass** das Fluoratom die Position 5 einnimmt.

11. Verfahren zur Herstellung von Verbindungen der Formel 1 nach Anspruch 1, **dadurch gekennzeichnet, dass** man 3-Fluorbrenzkatechin mit 2,3-Dibrompropionamid umsetzt und das erhaltene 5-Fiuorbenzodioxan-2-carboxamid-Derivat kristallisiert, welches anschließend zum Nitril dehydratisiert wird, dann durch NBS bromiert wird, einer Behandlung mit einem Natriumalkoholat unterzogen wird, um das Imidat-Zwischenprodukt zu bilden, das anschließend mit Ethylendiamin in einem Alkohol behandelt wird.

12. Verfahren zur Herstellung von Verbindungen der Formel 1 nach Anspruch 1, **dadurch gekennzeichnet, dass** man das 6- oder 7-Fluor-2,3-dihydrobenzo-[1,4]dioxin-2-yl)methanol in ein Nitril überführt und dieses Nitril gemäß dem Verfahren von Anspruch 11 behandelt.

13. Verbindung der Formel 1 gemäß einem der Ansprüche 1 bis 10 als Medikament.

14. Pharmazeutische Zusammensetzungen, **dadurch gekennzeichnet, dass** sie mindestens eine Verbindung der Formel 1 gemäß den Ansprüchen 1 bis 10 und einen geeigneten Träger umfassen.

15. Verwendung einer Verbindung der Formel 1 nach einem der Ansprüche 1 bis 10 für die Herstellung eines Medikaments als Antagonist der adrenergen α₂-Rezeptoren, das als solcher zur Behandlung von neurodegenerativen Erkrankungen und ihrem Fortschreiten verwendet wird und bestimmt ist.

16. Verwendung einer Verbindung der Formel 1 nach einem der Ansprüche 1 bis 10 für die Herstellung eines Medikaments als Antagonist der adrenergen α₂-Rezeptoren, das als solcher zur Behandlung von kognitiven und Gedächtnisstörungen sowie Aufmerksamkeits- und Vigilanzdefiziten verwendet wird und bestimmt ist.

17. Verwendung einer Verbindung der Formel 1 nach einem der Ansprüche 1 bis 10 für die Herstellung eines Medikaments als Antagonist der adrenergen α₂-Rezeptoren, das als solcher zur Behandlung der Alzheimer-Krankheit verwendet wird und bestimmt ist.

18. Verwendung einer Verbindung der Formel 1 nach einem der Ansprüche 1 bis 10 für die Herstellung eines Medikaments als Antagonist der adrenergen α₂-Rezeptoren, das als solcher zur Behandlung der Parkinson-Krankheit, der Chorea Huntington, der amyotrophen Lateralsklerose, der Creutzfeldt-Jakob-Krankheit, der progressiven supranukleären Paralyse sowie des Fortschreitens dieser Krankheiten oder dieser Störungen verwendet wird und bestimmt ist.

19. Verwendung einer Verbindung der Formel 1 nach einem der Ansprüche 1 bis 10 für die Herstellung eines Medikaments als Antagonist der adrenergen α₂-Rezeptoren, das als solcher zur Behandlung von Pathologien, die Zerebralattacken, ischämische Störungen, vaskuläre zerebrale Krankheitserscheinungen und deren Konsequenzen betreffen, sowie von Depression, Narkolepsie, männlichen sexuellen Dysfunktionen, von Störungen, die mit dem erworbenen Immunodefizienzsyndrom verbunden sind, sowie dem Fortschreiten dieser Krankheiten oder dieser Störungen verwendet wird und bestimmt ist.

## Claims

1. Compounds corresponding to the structure of general formula 1: 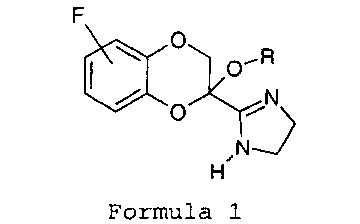 in which:
- R represents a linear, branched or cyclized alkyl or alkenyl group containing 1 to 7 carbon atoms, or a benzyl group, and
- the fluorine atom can occupy position 5, 6, 7 or 8,
in their racemic form and their dextrorotatory and levorotatory pure enantiomeric forms, and also the addition salts thereof.

2. Compounds of formula 1 according to Claim 1, **characterized in that** the radical R is a methyl group.

3. Compounds of formula 1 according to Claim 1, **characterized in that** the radical R is an ethyl group.

4. Compounds of formula 1 according to Claim 1, **characterized in that** the radical R is an n-propyl group.

5. Compounds of formula 1 according to Claim 1, **characterized in that** the radical R is a isopropyl group.

6. Compounds of formula 1 according to Claim 1, **characterized in that** the radical R is an isobutyl group.

7. Compounds of formula 1 according to Claim 1, **characterized in that** the radical R is a cyclopropylmethyl group.

8. Compounds of formula 1 according to Claim 1, **characterized in that** the radical R is a allyl group.

9. Compounds of formula 1 according to Claim 1, **characterized in that** the radical R is a benzyl group.

10. Compounds according to one of Claims 1 to 9, **characterized in that** the fluorine atom occupies position 5.

11. Process for preparing the compounds of formula 1 according to Claim 1, **characterized in that** 3-fluorocatechol is reacted with 2,3-dibromopropionamide, and the 5-fluorobenzodioxane-2-carboxamide derivative obtained is crystallized, and is then dehydrated into nitrile, and then brominated with NBS, subjected to a treatment with a sodium alkoxide to form the intermediate imidate, which is then treated with ethylenediamine in an alcohol.

12. Process for preparing the compounds of formula 1 according to Claim 1, **characterized in that** 6- or 7-fluoro-2,3-dihydro-benzo[1,4]dioxin-2-yl)methanol is converted into nitrile, and this nitrile is treated according to the process of Claim 11.

13. As a medicinal product, a compound of formula 1 according to one of Claims 1 to 10.

14. Pharmaceutical compositions, **characterized in that** they comprise at least one compound of formula 1 according to Claims 1 to 10, and a suitable excipient.

15. Use of a compound of formula 1 according to one of Claims 1 to 10, for the preparation of a medicinal product as an α₂-adrenergic receptor antagonist used and intended in this respect to treat neurodegenerative diseases and their progression.

16. Use of a compound of formula 1 according to one of Claims 1 to 10, for the preparation of a medicinal product as an α₂-adrenergic receptor antagonist used and intended in this respect to treat cognitive and memory disorders, and also attention deficit and vigilance deficit.

17. Use of a compound of formula 1 according to one of Claims 1 to 10, for the preparation of a medicinal product as an α₂-adrenergic receptor antagonist used and intended in this respect to treat Alzheimer's disease.

18. Use of a compound of formula 1 according to one of Claims 1 to 10, for the preparation of a medicinal product as an α₂-adrenergic receptor antagonist used and intended in this respect to treat Parkinson's disease, Huntington's chorea, amyotrophic lateral sclerosis, Creutzfeld-Jacob disease, progressive supranuclear paralysis, and also the evolution of these diseases or disorders.

19. Use of a compound of formula 1 according to one of Claims 1 to 10, for the preparation of a medicinal product as an α₂-adrenergic receptor antagonist used and intended in this respect to treat pathologies relating to cerebral attacks, ischemic disorders, cerebrovascular accidents and their consequences, and also depression, narcolepsy, male sexual dysfunctions, disorders associated with acquired immunodeficiency syndrome, and also the evolution of these diseases or disorders.
